# FASCICULE DE BREVET EUROPEEN

(11) **EP 0 973 650 B1**
(45) Date de publication et mention de la délivrance du brevet: **09.04.2003**
(21) Numéro de dépôt: 98920602.4
(22) Date de dépôt: 09.04.1998
(51) Int. Cl.: B44C 1/165, B44C 1/175, A45D 37/00, A45D 34/04, A61M 35/00, A61M 37/00, B65D 75/58

(54) **DISPOSITIF POUR DIFFUSER UNE OU PLUSIEURS DOSES DE PRODUIT FLUIDE**
VORRICHTUNG ZUR DOSIERTEN DIFFUSION VON EINER ODER MEHREREN STRÖMENDEN MEDIEN
DEVICE FOR DIFFUSING ONE OR SEVERAL FLUID PRODUCT DOSES

(30) Priorité: 10.04.1997 FR 9704390; 29.04.1997 FR 9705261; 14.05.1997 FR 9705896; 20.01.1998 FR 9800562; 29.01.1998 FR 9801003; 04.03.1998 FR 9802587
(43) Date de publication de la demande: 26.01.2000
(73) Titulaire: Martel, Stephane Christopher, 92250 La Garenne Colombes (FR); Cathala, Francois Henri, 75018 Paris (FR); Bochenek, Valerie Frederique, 80200 Doingt (FR); Lacombe-Gervaz, Valerie Christine, 92240 Malakoff (FR)
(72) Inventeur: Martel, Stephane Christopher, 92250 La Garenne Colombes (FR); Cathala, Francois Henri, 75018 Paris (FR); Bochenek, Valerie Frederique, 80200 Doingt (FR); Lacombe-Gervaz, Valerie Christine, 92240 Malakoff (FR)
(74) Mandataire: Mouget-Goniot, Claire
(86) Numéro de dépôt international: FR9800724
(87) Numéro de publication internationale: WO98045127

(56) Documents cités:
- EP-A- 0 294 189
- WO-A-96/32142
- WO-A-97/06073
- BE-A- 677 945
- DE-A- 19 606 839
- DE-U- 29 604 116
- FR-A- 2 632 936
- US-A- 3 826 259
- US-A- 3 998 559
- US-A- 4 044 181
- US-A- 4 084 910
- US-A- 4 291 697
- US-A- 4 430 013
- US-A- 4 522 864
- US-A- 4 665 901
- US-A- 4 812 067
- US-A- 5 090 832
- US-A- 5 578 353

## Description

La présente invention concerne un dispositif pour diffuser une ou plusieurs doses de produit fluide, tel qu'un produit liquide, crémeux, gélatineux ou gazeux, ainsi qu'un dispositif d'application de tatouage temporaire adhésif utilisant le dispositif de diffusion précité. Le dispositif de diffusion de l'invention s'applique notamment à la distribution de produit pharmaceutique, cosmétique, alimentaire, hygiénique, de parfumerie et d'entretien.

Dans le domaine de la parfumerie et de la cosmétique, il est courant de distribuer au public des échantillons gratuits contenant une petite dose de produit, pour faire la promotion du produit et pour satisfaire l'engouement du public pour ce type d'échantillons. Ces échantillons de produit sont généralement conditionnés dans de petits flacons ou des pochettes qui sont munis d'un bouchon amovible, d'un pulvérisateur, d'une languette déchirable, ou d'une ligne de cassure pour distribuer le produit. Ces moyens de distribution ont un coût de fabrication relativement élevé compte tenu de leur usage unique, ils sont difficiles à manipuler et présentent en outre un risque de blesser l'utilisateur.

Un dispositif connu de ce genre comporte un emballage plastique ou une plaquette alvéolaire définissant une ou plusieurs cavités contenant un produit généralement liquide, l'emballage ou la plaquette présentant une languette destinée à être cassée par pliage pour accéder à la cavité.

Un autre dispositif connu comporte une première pellicule plastique moulée définissant une cavité pour y recevoir le produit et une deuxième pellicule venant obturer ladite cavité, ladite deuxième pellicule pouvant être retirée ou déchirée, pour accéder à la cavité.

Les plaquettes alvéolaires de ce type sont également utilisées pour conditionner des médicaments sous la forme de pastilles ou de gélules ainsi que pour conditionner des lentilles de contact souples.

On connaît également dans le domaine alimentaire des pochettes plastiques déchirables destinées à contenir une dose de sauce tomate ou de sauce salade.

Dans l'hôtellerie, on utilise également de telles pochettes pour offrir à la clientèle des doses indépendantes de savon liquide ou de shampooing.

On notera également qu'un tel dispositif peut être utilisé pour contenir des doses désodorisantes pour diffuser dans l'air ambiant un produit gazeux parfumé contenu dans une pochette ou un flacon. On notera que le dispositif de l'invention peut être encarté dans un magazine.

Le document FR-A-2 632 936 décrit un dispositif de distribution de produit cosmétique liquide ou semi-liquide, ce dispositif étant constitué d'une pellicule relativement mince en matière plastique définissant un petit volume étanche dans lequel est conditionnée une dose de produit susceptible d'être libérée par écrasement dudit volume par pression des doigts. Toutefois, cette pellicule plastique de forme globalement sphérique éclate dans les doigts lors de son écrasement, ce qui rend son utilisation désagréable pour l'utilisateur qui aura les doigts imprégnés de produit.

Enfin, il a également déjà été proposé divers dispositifs pour diffuser des doses de produit liquide contenu dans un ou plusieurs compartiments dont la paroi comporte une zone d'affaiblissement constituée par exemple d'une zone de faible épaisseur, d'une encoche, d'une zone perforée ou d'une zone rigide cassable (brevets US 4,430,013, US 4,665,901, US4,291,697 et US 5,090,832 et demandes internationales WO 97/06073 et WO 96/32142) ou comportant un embout cassable (brevet US 3,826,259) permettant la diffusion du produit sous l'action d'une pression ou d'une pliure. L'invention a pour but d'éliminer les inconvénients précités et de proposer un dispositif pour diffuser une ou plusieurs doses de produit fluide, qui soit simple à utiliser, économique à fabriquer, efficace en fonctionnement, peu encombrant, plus léger et qui minimise les pertes de produits lors de la diffusion.

A cet effet, l'invention a pour objet un dispositif pour diffuser une ou plusieurs doses de produit fluide, chaque dose étant contenue dans une réserve étanche qui est définie par une enveloppe au moins partiellement en matière souple déformable, de façon qu'une pression d'écrasement (P) exercée sur la réserve provoque l'éclatement de l'enveloppe pour libérer le produit contenu dans la réserve, l'enveloppe étant pourvue localement d'au moins une zone d'affaiblissement formant une pré-ouverture, de façon à provoquer l'éclatement instantané de l'enveloppe au niveau de la pré-ouverture, le flux de produit (F) étant ainsi canalisé par la pré-ouverture dans une orientation prédéterminée, l'enveloppe comportant deux pellicules en matière plastique ou analogue, en métal, ou en matériaux composites, hermétiquement liées, par exemple par collage ou thermosoudage, l'une à l'autre dans une région périphérique pour définir entre elles la réserve contenant le produit, caractérisé par le fait que la région périphérique de liaison comporte localement au moins une zone de thermosoudure fragilisée formant la préouverture précitée, alors que le reste de la région périphérique de liaison est une thermosoudure définitive.

Grâce à l'invention, la diffusion du produit ne s'effectue plus directement en contact avec les doigts, ce qui évite de salir les doigts et supprime les risques de blessure. En outre, comme le flux de produit est dirigé dans une orientation sélectionnée, on peut optimiser l'utilisation du produit à distribuer, sans perte inutile de produit. La diffusion du produit s'effectuant par simple pression sur le dispositif, l'utilisation en sera facilitée et accélérée.

Avantageusement, la zone de thermosoudure fragilisée est définie entre une portion périphérique en forme de languette de l'une des pellicules et une portion correspondante de l'autre pellicule, lesdites portions étant superposées et s'étendant sensiblement à distance du bord périphérique de la réserve.

Dans une première variante, la languette précitée fait saillie à l'intérieur de la réserve et est thermosoudée de manière détachable, à une portion correspondante de l'autre pellicule, qui présente un orifice pour diffuser le produit à l'extérieur, lorsque la languette s'est détachée sous l'effet d'une pression d'écrasement de la réserve.

Dans une autre variante, la languette fait saillie à l'extérieur de la réserve et est thermosoudée de manière détachable à une portion correspondante de l'autre pellicule, à l'exception de deux bords latéraux de la languette et, le cas échéant, de l'extrémité distale de la languette, qui sont thermosoudés de manière définitive à l'autre pellicule pour définir au moins un pré-canal d'ouverture de la réserve.

Dans une première forme de réalisation, le bord d'extrémité distale de la languette est soudé de manière détachable pour définir au moins un précanal ouvrable en bout pour la sortie du produit. Dans ce cas, la languette peut se prolonger au-delà de la portion correspondante de l'autre pellicule, de manière à pouvoir replier et fixer de manière détachable la portion saillante de la languette à l'autre pellicule, afin d'isoler le passage de sortie du produit des éléments contaminants extérieurs, avant son utilisation.

Dans une autre forme de réalisation, le bord d'extrémité distale de la languette est thermosoudé de manière définitive à l'autre pellicule, pour définir au moins un précanal fermé, et au moins un orifice est ménagé à travers la languette et/ou la portion correspondante de l'autre pellicule pour la sortie du produit, lorsque la thermosoudure fragilisée se sera détachée sous l'effet de la pression exercée sur la réserve.

Selon une autre caractéristique, chaque orifice de sortie peut comporter un embout saillant pour définir une buse de projection du produit.

Selon encore une autre caractéristique, chaque orifice peut être recouvert d'une pellicule de protection escamotable sous l'effet de la pression du produit sortant par l'orifice, afin d'isoler l'orifice des éléments contaminants extérieurs avant l'utilisation.

Avantageusement, l'orifice de sortie peut être constitué d'une pluralité de micro-orifices, disposés par exemple en étoile, pour pulvériser le produit à sa sortie.

Selon encore une autre caractéristique, la languette comporte au moins une ligne intermédiaire de soudure définitive avec l'autre pellicule entre ses bords latéraux thermosoudés de manière définitive, ladite ligne de soudure intermédiaire étant sensiblement parallèle auxdits bords latéraux pour définir une pluralité de précanaux de diffusion du produit.

Dans une forme particulière de réalisation du dispositif, la réserve se présente sous la forme générale d'une bulle creuse contenant le produit à diffuser.

Dans une autre forme de réalisation, les deux pellicules formant la réserve sont sensiblement planes et superposées, au moins l'une d'entre elles comportant une région bombée vers l'extérieur, obtenue par exemple par moulage, pour définir la réserve précitée avec l'autre pellicule, la région périphérique des pellicules autour de ladite région bombée s'étendant sensiblement dans un même plan pour définir une surface périphérique de support et de préhension du dispositif.

Dans une variante, les deux pellicules comportent une ligne de pliage commune passant au droit de la préouverture de la réserve, de façon qu'en exerçant une pression sur la surface périphérique de support du dispositif, de part et d'autre de la ligne commune de pliage, la réserve soit écrasée par pincement entre les deux parties repliées du dispositif, ce qui provoque la diffusion du produit par la préouverture située sur l'arête du dièdre formé par les deux parties repliées du dispositif.

Selon une autre caractéristique, la pellicule qui est munie d'une préouverture comporte sur sa face externe une couche de matière formant un tampon applicateur, qui est hermétiquement lié à la périphérie de ladite surface de support, de manière à imprégner le tampon applicateur avec le produit lors de l'éclatement de la réserve par la préouverture, le tampon applicateur servant à appliquer le produit sur toute surface choisie, l'applicateur pouvant être un papier absorbant, une matière textile, spongieuse ou cotonneuse. Dans ce cas, on peut prévoir un espace libre intercalaire entre le tampon applicateur et la pellicule en vis-à-vis pour permettre au produit libéré par la réserve de se répartir de manière homogène sur toute la surface du tampon applicateur.

Avantageusement, on prévoit un film protecteur escamotable lié à l'un des bords du tampon applicateur sur sa face externe, pour le recouvrir et l'isoler de tout élément extérieur pouvant le détériorer et/ou le contaminer.

La réserve peut contenir un produit liquide, crémeux, gélatineux ou gazeux.

Le dispositif conforme à l'invention peut notamment être un dispositif d'application d'un tatouage temporaire adhésif.

On connaît par le brevet US N° 5578353 un dispositif de décalcomanie d'un ticket portant une encre, à l'aide d'une éponge mouillée ou d'une pulvérisation appliquée sur la · peau. Un tel dispositif nécessite toutefois l'utilisation d'une éponge mouillée ou d'un autre instrument équivalent, ce qui complique l'utilisation.

Le document DE-A-19 606 839 décrit un dispositif comportant un support auxiliaire, une couche d'adhésif de contact, une couche de transfert entre le support et la couche d'adhésif, ladite couche de transfert contenant un liant qui adhère plus fortement à la couche adhésive qu'au support, la couche de transfert et/ou la couche d'adhésif comportant, d'une part, de fines particules creuses contenant de l'eau et, d'autre part un alcool solide soluble dans l'eau, de façon qu'une pression exercée sur le support provoque l'éclatement des microparticules pour appliquer ladite couche de transfert sur une surface. Toutefois, un tel dispositif nécessite de fabriquer des microsphères dans une couche de polymère opaque, ladite couche de polymère opaque étant destinée à être transférée sur la surface, ce qui complique la fabrication et augmente le coût de production.

L'invention a pour deuxième but d'éliminer les inconvénients précités des dispositifs de tatouage temporaire adhésif antérieurs, et de proposer un dispositif d'application de tatouage temporaire adhésif qui soit simple et peu coûteux à fabriquer, et qui permette une application instantanée du tatouage temporaire adhésif sur toutes surfaces choisies.

A cet effet, l'invention concerne également un dispositif d'application d'un tatouage temporaire adhésif, utilisant le dispositif de diffusion défini précédemment, caractérisé en ce qu'une encre pour tatouage temporaire adhésif est positionnée sur la face externe du tampon applicateur, de sorte qu'une pression exercée par l'utilisateur sur la réserve provoque l'éclatement de l'enveloppe par la préouverture, ce qui génère l'humidification du tampon applicateur et le transfert instantané du tatouage temporaire adhésif sur la surface choisie.

Avantageusement, le dispositif comporte un film protecteur escamotable lié à l'un des bords du tampon applicateur sur sa face externe, pour recouvrir l'encre de tatouage temporaire adhésif et l'isoler des éléments extérieurs pouvant la contaminer et/ou l'endommager. Le film protecteur peut être muni sur sa face externe d'une couche de matière adhésive recouverte d'une pellicule de protection pelable de façon à permettre de transférer le tatouage, déjà appliqué sur une surface, sur ladite couche de matière adhésive, après avoir pelé la pellicule de protection, et d'appliquer à nouveau ledit tatouage sur une autre surface.

Selon une autre caractéristique, le dispositif comporte une pellicule supplémentaire hermétiquement liée à la périphérie de la surface de support, du côté opposé à la préouverture, de façon à définir un volume d'air entre ladite pellicule supplémentaire et la réserve, ladite pellicule supplémentaire comportant localement une zone périphérique de liaison affaiblie, de façon qu'une pression d'écrasement exercée sur ladite pellicule supplémentaire provoque successivement ou simultanément l'éclatement sonore du volume d'air au niveau de la zone de liaison affaiblie et l'éclatement de la réserve par la préouverture.

L'invention sera mieux comprise, et d'autres buts, détails, caractéristiques et avantages de celle-ci apparaîtront plus clairement au cours de la description explicative détaillée qui va suivre de plusieurs modes de réalisation particuliers actuellement préférés de l'invention, donnés uniquement à titre illustratif et non limitatif, en référence aux dessins schématiques annexés, dans lesquels :
- la figure 1 est vue schématique en coupe transversale d'un premier mode de réalisation du dispositif de diffusion conforme à l'invention, comportant une préouverture au centre de l'une des pellicules définissant la réserve ;
- la figure 1A est une vue de dessous du dispositif de la figure 1, la pellicule munie de la préouverture étant omise ;
- la figure 2 est une vue analogue à la figure 1, le dispositif étant équipé, en outre, d'un tampon applicateur disposé en vis-à-vis de la préouverture,
- la figure 2A est une vue de dessus de la figure 2, montrant le tampon applicateur seul ;
- la figure 3 est une variante de réalisation du dispositif de la figure 2, le tampon applicateur étant en outre muni d'un film protecteur escamotable ;
- la figure 3A est une vue de dessous de la figure 3, le tampon applicateur étant omis ;
- la figure 4 est une vue en plan de dessus d'un autre mode de réalisation du dispositif de diffusion selon l'invention, la zone de préouverture étant définie sur un bord périphérique interne de la réserve ;
- la figure 5 est une vue analogue à la figure 4, la réserve ne comportant pas de support périphérique environnant ;
- la figure 6 est une variante de réalisation du dispositif de la figure 4, la zone de préouverture étant définie sur un bord périphérique externe de la réserve ;
- la figure 6A est une vue en coupe transversale suivant la ligne VI de la figure 6 ;
- la figure 7 est une vue analogue à la figure 6A, mais représentant la réserve dépourvue de support périphérique environnant ;
- la figure 8 est une vue de dessus d'un dispositif similaire à celui de la figure 7, mais représentant une réserve en forme générale de bulle sphérique ;
- la figure 8A est une vue analogue à la figure 8, mettant en évidence le détachement de la soudure fragilisée du dispositif lors de son utilisation;
- les figures 9 et 9A sont des vues en coupe suivant la ligne IX respectivement des figures 8 et 8A ;
- les figures 10 et 10A sont des vues respectivement similaires aux figures 8 et 9, mais représentant une variante de réalisation du dispositif ;
- la figure 11 est une vue partielle et agrandie d'une zone thermosoudée fragilisée intégrant une pluralité de micro-orifices pouvant être utilisée dans les modes de réalisation des figures 4 à 10 ;
- les figures 12, 12A, 13, 13A sont des vues similaires respectivement aux figures 8, 8A, 9, 9A, mais représentent une variante de réalisation de l'invention ;
- les figures 14, 14A, 15 et 15A sont des vues similaires respectivement aux figures 12, 12A, 13 et 13A, mais représentent encore une autre variante de réalisation ;
- les figures 16 et 16A sont des vues respectivement de dessus et de dessous d'un dispositif du type de la figure 1, dans lequel une ligne de pliage commune est formée sur les deux pellicules au droit de la préouverture ;
- la figure 17 est une vue latérale du dispositif de la figure 16, montrant la diffusion du produit sur une surface choisie, dans une orientation sélectionnée ;
- la figure 17A est une vue analogue de la figure 17, le dispositif étant équipé en outre d'un tampon applicateur ;
- la figure 18 montre une réserve sous la forme d'une bulle sphérique présentant une préouverture constituée d'une zone de plus faible épaisseur ;
- les figures 18A et 18B représentent la bulle de la figure 18, lors de deux étapes successives d'utilisation ;
- les figures 19A à 19H sont des vues schématiques de dessus représentant diverses formes de réalisation de la réserve et du support environnant d'un dispositif conforme à l'invention ;
- la figure 20 est une vue analogue à la figure 3, mais représentant un dispositif d'application de tatouage temporaire adhésif comportant en outre une couche d'encre adhésive sur le tampon applicateur ; et
- la figure 21 est une vue analogue à la figure 2, mais représentant une variante de réalisation de dispositif d'application de tatouage temporaire adhésif, comportant en outre une couche d'encre adhésive, un film protecteur muni d'une pellicule pelable, et une pellicule supplémentaire au dessus de la réserve pour engendrer un bruit d'éclatement ; étant entendu que les dispositifs illustrés par les figures 1-3A ; 16-18B ; 20 et 21 correspondent à des modes de réalisation de l'invention qui ne sont pas couverts par les revendications.

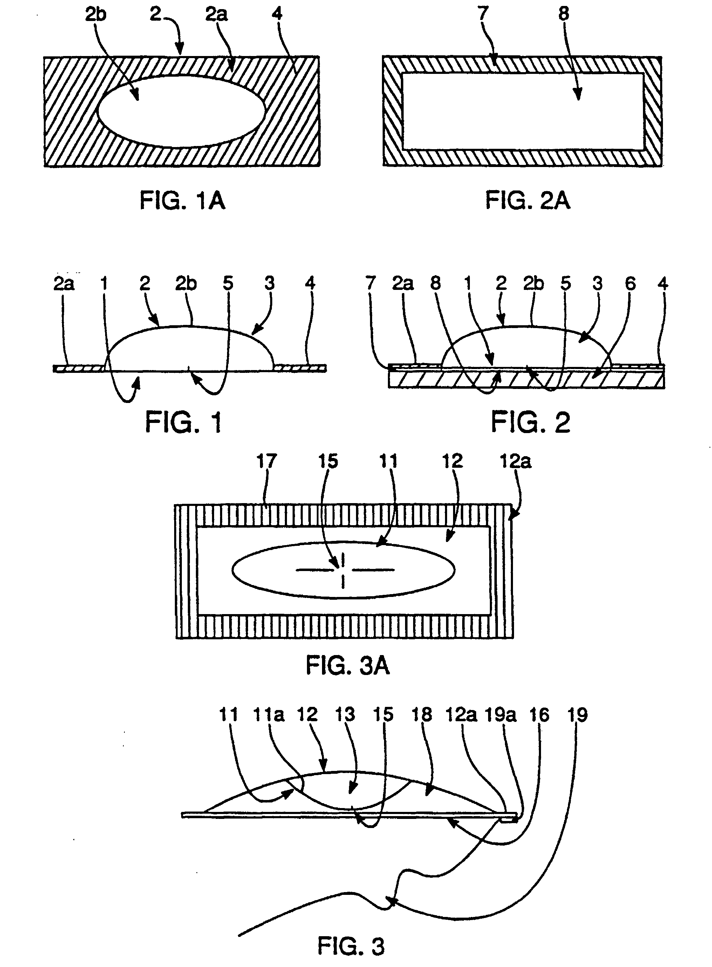

Sur la figure 1, on voit en coupe une première pellicule sensiblement plane 1, sur laquelle est appliquée une deuxième pellicule 2 présentant une région périphérique 2a qui est collée ou thermosoudée à une région périphérique correspondante de la pellicule 1 et une région centrale bombée vers l'extérieur 2b, obtenue par exemple par moulage, de manière à définir entre ladite zone centrale bombée 2b et la pellicule 1 en vis-à-vis un espace clos 3 définissant une réserve pour un produit à distribuer. On a indiqué par des traits hachurés la zone de collage ou de thermosoudage 4. La pellicule inférieure 1 comporte en son centre une préouverture définie par une prédécoupe en forme de croix 5 pour la diffusion du produit vers l'extérieur. On voit sur la figure 1A, que la région périphérique 2a de la pellicule 2 est hachurée pour indiquer l'emplacement du collage ou du thermosoudage 4. La forme générale du dispositif est sensiblement rectangulaire, alors que la réserve 3 a une forme globalement en ellipse en vue de dessus.

La région périphérique 2a de la pellicule 2 forme avec la portion correspondante de la pellicule 1 un support annulaire servant notamment pour la préhension du dispositif.

En se référant maintenant à la figure 2, on voit qu'une couche de matière 6 formant un tampon applicateur est périphériquement fixée à la surface externe de la pellicule 1, par l'intermédiaire d'un cordon de colle ou de soudure 7, ce dernier délimitant un espace intercalaire 8 entre la préouverture 5 de la pellicule 1 et la surface interne en vis-à-vis du tampon applicateur 6. Ce tampon applicateur 6 peut être un papier absorbant, une matière textile, spongieuse ou cotonneuse. L'espace intercalaire 8 a pour but de permettre la diffusion du produit contenu dans la réserve 3 et sortant par la préouverture 5 sur sensiblement toute la surface interne du tampon applicateur 6, pour permettre son imprégnation en produit de manière sensiblement homogène. Ainsi, le produit pourra être appliqué sur toute surface choisie par l'intermédiaire de la surface externe du tampon applicateur 6.

Bien entendu, les liaisons 4 et 7 sont hermétiques au produit à distribuer.

En se référant à la figure 3, on voit une variante de réalisation dans laquelle la pellicule supérieure 12 présente une forme légèrement concave vers le bas et la pellicule 11 épouse sensiblement la forme de la pellicule 12, à l'exception de la portion centrale 11a qui est bombée vers le bas et munie en son centre de la préouverture 15. La réserve 13 est donc ici tournée vers le bas, contrairement à la figure 1 où la réserve 3 était tournée vers le haut.

Les pellicules 11 et 12 sont reliées à leur, périphérie 12a à un tampon applicateur 16, par un cordon périphérique de colle ou de soudure 17 (voir figure 3A). Le tampon applicateur 16 est relié par un bord transversal à une patte 19a d'un film protecteur flexible transparent 19 qui est destiné à recouvrir la surface externe du tampon applicateur 16 pour l'isoler de l'extérieur et le protéger de toute contamination et/ou détérioration.

On voit sur la figure 3 que l'espace intercalaire 18 prévu entre la réserve 13 et le tampon applicateur 16 est nettement plus important que l'espace intercalaire 8 de la figure 2, car ici la surface bombée 11a de la réserve est tournée vers le tampon applicateur.

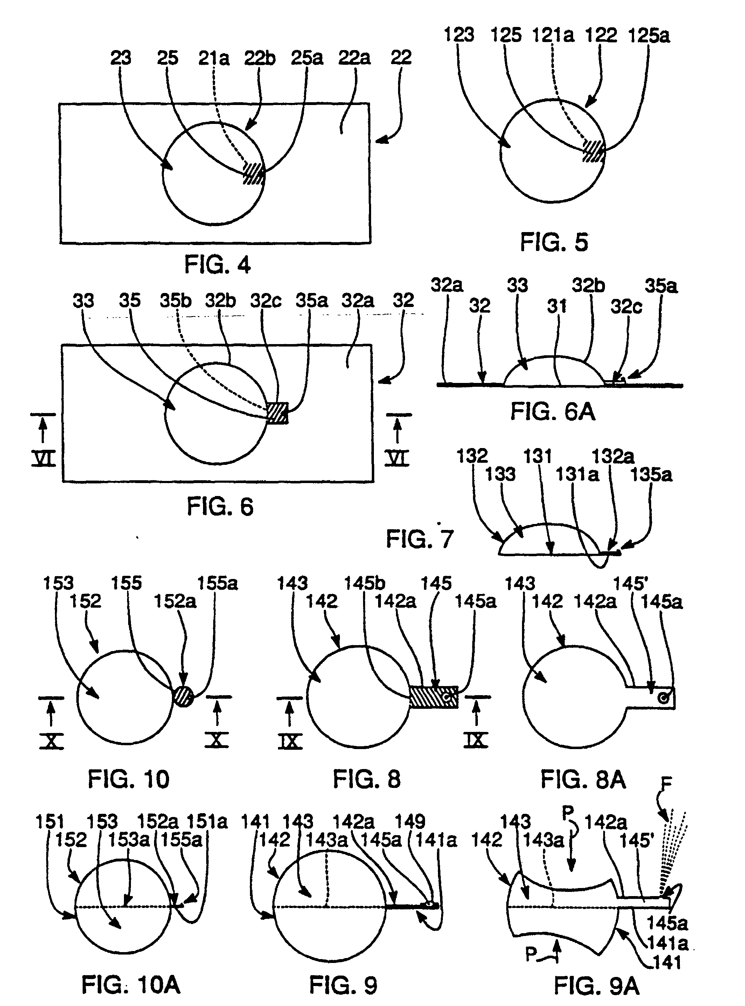

Sur la variante de la figure 4, la pellicule supérieure 22 présente également une forme rectangulaire avec une portion centrale 22b sensiblement en forme de calotte sphérique bombée vers l'extérieur et une région périphérique plane 22a. La portion centrale 22b de la pellicule 22 définit avec la pellicule inférieure (non représentée) une réserve 23.

La pellicule inférieure comporte une languette 21a recourbée vers l'intérieur, qui est collée ou thermosoudée, comme indiquée par la zone hachurée 25, à la surface interne de la portion bombée 22b de la pellicule 22. Cette languette 21a est hermétiquement fixée par des points de colle détachables ou une thermosoudure fragilisée à la portion 22b de la pellicule 22, pour permettre à cette languette 21a de se détacher, lorsqu'une pression est exercée sur la réserve 23. Lorsque la languette 21a est détachée, le produit peut diffuser vers l'extérieur par un orifice 25a ménagé à travers la portion 22b de la pellicule 22, au droit de la languette 21a. Avant qu'une pression soit exercée sur la réserve 23, l'orifice 25a est hermétiquement obturé par la liaison affaiblie 25 avec la languette 21a.

On pourrait en variante, prévoir l'orifice 25a sur la pellicule inférieure et la languette 21a sur la pellicule supérieure 22.

Le seul bord de la languette 21a qui n'est pas détachable est celui qui est commun avec le bord périphérique de la portion 22b de la pellicule 22 qui est thermosoudée de manière définitive avec la pellicule inférieure.

Dans la variante de la figure 6, la pellicule supérieure 32 comporte également une portion centrale 32b en forme de calotte sphérique et une portion plane environnante 32a. La réserve 33 est définie entre la portion centrale bombée 32b de la pellicule 32 et la surface interne en vis-à-vis de la pellicule plane inférieure 31 (voir figure 6A).

La zone de préouverture (représentée hachurée) est définie ici par une languette 32c faisant partie de la portion périphérique 32a de la pellicule 32, ladite languette 32c s'étendant à partir d'un bord périphérique de la portion centrale 32b et étant thermosoudée de manière définitive par tous ces côtés à la pellicule inférieure 31, à l'exception de son bord 35b, commun avec le bord périphérique de la portion centrale 32b, qui est relié par une soudure fragilisée à la pellicule inférieure 31. La languette 32c est également reliée par une thermosoudure fragilisée 35 sur toute sa surface à la pellicule inférieure 31, pour la raison expliquée ci-après. Un orifice 35a est ménagé à travers la languette 32c pour permettre la diffusion du produit vers l'extérieur lorsque le bord interne 35b et toute la surface interne 35 de la languette 32c s'est détachée de la pellicule inférieure 31, sous l'effet d'une pression exercée sur la réserve 33. La zone de préouverture 35 forme ainsi un pré-canal de sortie du fluide, lequel pré-canal se dilate en un canal en détachant la soudure fragilisée sous l'effet de la pression, ce qui permet au fluide contenu dans la réserve 33 de passer dans le canal vers l'orifice de sortie 35a.

Sur la figure 7, les deux pellicules 131 et 132 ne comportent pas de région périphérique plane, mais se raccordent l'une à l'autre uniquement par leurs bords périphériques communs. Autrement dit, la pellicule 132 présente une forme générale en calotte sphérique et la pellicule 131 présente une forme sensiblement en disque plan. La pellicule 132 comporte en outre une languette 132a qui fait saillie vers l'extérieur et qui se superpose à une languette correspondante 131a qui fait saillie vers l'extérieur de la pellicule 131. La languette 132a comporte un orifice ou une buse de projection saillante 135a pour le produit contenu dans la réserve 133, les languettes 131a et 132a étant liées par une soudure fragilisée, à l'exception de leurs bords externes qui sont mutuellement soudés de manière définitive.

Sur la figure 5, on a présenté le même dispositif que sur la figure 4, sauf qu'il ne comporte pas ici de surface périphérique plane entourant la réserve 123. La pellicule supérieure 122 présente une forme générale en calotte sphérique, de manière analogue à la pellicule 132 de la figure 7. Au voisinage de son bord périphérique, la pellicule 122 comporte un orifice 125a qui est obturé par une languette interne 121a de la pellicule inférieure (non représentée) avec une soudure fragilisée 125. Le fonctionnement du dispositif de la figure 5 est identique à celui de la figure 4.

En variante, l'élément 22b, 122, 32b et 132 respectivement des figures 4 à 7 peut être constitué d'une coque rigide remplaçant la pellicule souple moulée, ce qui permet d'encarter le dispositif entre les feuilles d'une revue ou d'un magazine en le plaçant par la pellicule 21, 121, 31 et 131 sur une feuille avec un adhésif détachable. Ainsi, le dispositif ne risque pas d'éclater sous l'effet de la pression du magazine, lors de son transport et de son stockage, et on peut détacher le dispositif pour le repositionner sur un autre support ou une autre surface choisie ou pour le faire éclater en exerçant une pression sur la pellicule plane souple.

Dans le mode de réalisation représenté sur les figures 8 et 9, la réserve 143 a une forme générale sphérique obtenue par l'assemblage de deux pellicules hémisphériques 141 et 142 le long de leur bord équatorial commun 143a. Les pellicules 141 et 142 comportent chacune une languette respective 141a et 142a qui sont superposées et font saillie vers l'extérieur. Ces languettes 141a et 142a sont liées l'une à l'autre sur tous leurs côtés par une soudure permanente, à l'exception de leur bord interne 145b et de leur surface en regard 145 qui sont liés par une soudure fragilisée, comme indiqué dans la zone hachurée. La languette 142a et/ou 141a comportent un orifice 145a débouchant dans la zone de soudure fragilisée 145, pour permettre l'éjection du produit contenu dans la réserve 143, lorsque la zone de soudure fragilisée 145 s'est détachée, formant un canal d'éjection 145' comme illustré sur les figures 8A et 9A. En effet, sous l'effet d'une force de pression exercée sur chaque pellicule, comme illustré par les flèches P sur la figure 9A, la zone de soudure fragilisée 145 se détache formant le canal 145' ce qui permet l'éjection par l'orifice 145a du fluide comme indiqué par la flèche F.

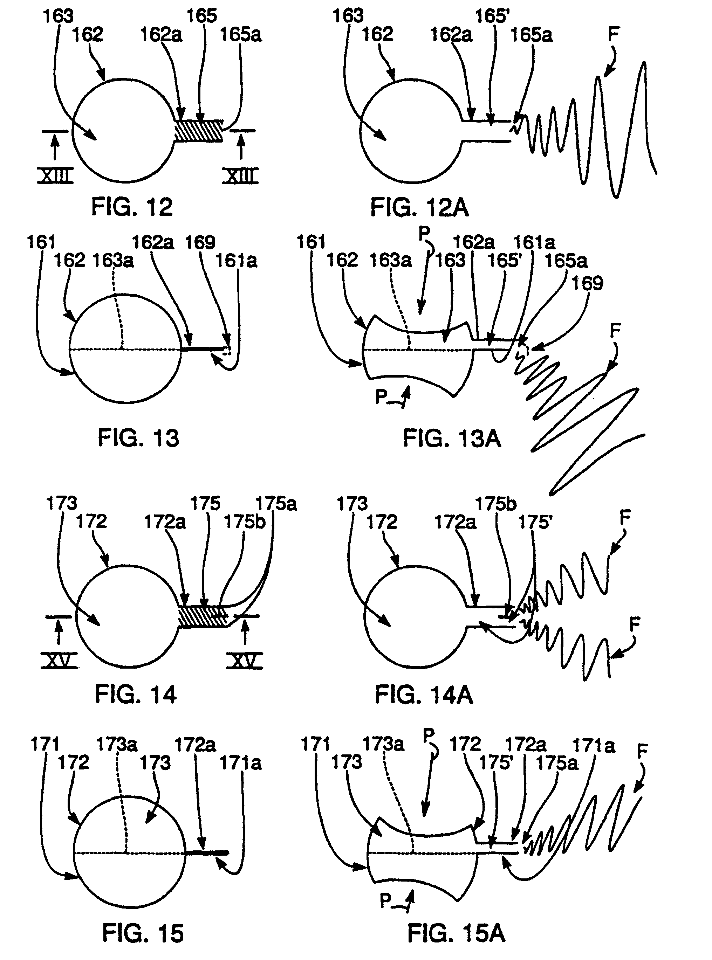

Bien entendu, la languette 141a et/ou 142a peuvent avoir plusieurs orifices d'éjection de produit, comme illustré sur la figure 11. Sur cette figure, plusieurs orifices 146 disposés en étoile débouchent dans la zone de soudure fragilisée 145 pour avoir un effet de pulvérisation ou micro-jets du produit contenu dans la réserve 143.

Sur la figure 9, une petite pellicule supplémentaire de protection 149 est collée de manière détachable ou liée par une thermosoudure fragilisée sur l'orifice 145a pour le recouvrir et l'isoler des éléments contaminants ou détériorant extérieurs, notamment pour des régions d'hygiène.

Sur les figures 10 et 10A, les éléments qui sont identiques ou similaires à ceux illustrés sur les figures 8 et 9 portent les mêmes chiffres de référence augmentés d'une dizaine. La variante de la figure 10 diffère du mode de réalisation des figures 8 et 9, principalement par le fait que les languettes 152a et 151a ont ici une forme générale circulaire, au lieu de la forme sensiblement rectangulaire des figures 8 et 9, et par le fait que l'orifice de sortie 155a présente ici une forme de buse saillante vers l'extérieur au lieu du simple orifice 145a des figures 8 et 9.

Sur les figures 12 et 13, les éléments identiques ou similaires à ceux illustrés sur les figures 8 et 9 portent les mêmes chiffres de référence augmentés d'une vingtaine. La variante des figures 12 et 13 diffère du mode de réalisation des figures 8 et 9, essentiellement par le fait que l'orifice de sortie du fluide est constitué ici par le bord distal 165a de la zone de soudure fragilisée 165 entre les languettes 162a et 161a. Dès lors, lorsque l'on exerce une pression P sur la réserve 163, les languettes 161a et 162a se détachent, définissant un canal d'évacuation 165' qui débouche en tête 165a vers l'extérieur, pour éjecter le produit comme indiqué par la flèche F. Contrairement au mode de réalisation des figures 8 et 9, le fluide F est sur les figures 12 et 13 diffusé vers l'extérieur sans être pulvérisé. Il convient bien par exemple pour un produit crémeux, gélatineux ou pâteux.

L'orifice distal de sortie 165a sur la figure 13 est recouvert par un prolongement 169 de la languette 162a de manière à pouvoir replier et fixer de manière détachable la portion saillante de la languette à l'autre pellicule, pour l'isoler des éléments contaminants et/ou détériorants de l'extérieur, notamment pour des raisons d'hygiène. Sous l'effet du fluide sous pression éjecté par le canal ouvert 165', le prolongement 169 de la languette 162a se détache, comme illustré sur la figure 13A, et contribue également à orienter le flux de produit F dans une direction déterminée. Le prolongement 169 pourrait également servir d'applicateur.

Sur les figures 14 et 15, les éléments identiques ou similaires à ceux du mode de réalisation des figures 12 et 13, portent les mêmes chiffres de référence augmentés d'une dizaine. La variante des figures 14 et 15 diffère essentiellement du mode de réalisation des figures 12 et 13 par le fait que le dispositif des figures 14 et 15 comporte une ligne de soudure permanente intermédiaire 175b dans la zone de soudure fragilisée 175, ladite ligne de soudure permanente intermédiaire 175b s'étendant entre les bords latéraux des languettes 171a et 172a et parallèlement à ces derniers, de manière à définir deux canaux parallèles 175' plus étroits débouchant à l'extrémité distale 175a sur l'extérieur, afin d'obtenir deux jets séparés de fluide F. Bien entendu, on pourrait prévoir plusieurs lignes intermédiaires de soudure permanente pour obtenir plusieurs jets de fluide.

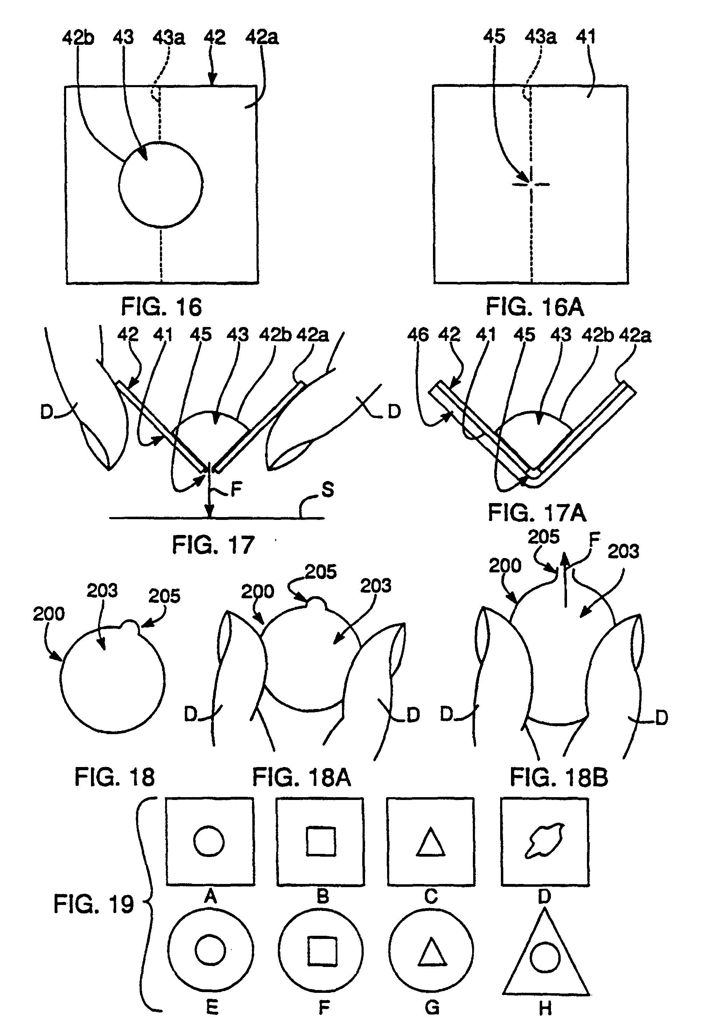

Dans un autre mode de réalisation de la figure 2 représenté sur les figures 16, 16A et 17, le dispositif comporte une pellicule supérieure 42 dont une portion centrale circulaire 42b est bombée vers l'extérieur et se raccorde à une portion périphérique plane environnante 42a, et une pellicule inférieure plane de forme carrée 41 qui est fixée de manière permanente à la portion plane périphérique 42a de la pellicule 42. Au droit de la portion circulaire bombée 42b de la pellicule 42, la pellicule 41 comporte une prédécoupe en forme de croix 45, de préférence au centre de ladite portion circulaire 42b. La réserve 43 est définie entre la portion bombée 42b et la pellicule inférieure sous-jacente 41. La pellicule 41 et la région périphérique plane 42a de la pellicule 42 comportent en outre une ligne de pliage commune 43a qui s'étend sur toute leur longueur et passe par la préouverture 45, ce qui partage le dispositif en deux parties pliables. Ainsi, en exerçant une pression avec les doigts D (voir figure 17) sur chaque partie pliable du dispositif, on comprime la réserve 43 entre les deux parties pliées du dispositif, ce qui provoque l'éclatement de la réserve par la préouverture 45 pour éjecter le produit dans la direction de la flèche F, vers une surface choisie S. Dans ces conditions, on n' agit plus directement sur la réserve 43, mais sur les parties périphériques des pellicules 41 et 42.

Sur la figure 17A, un tampon applicateur 46 est fixé sur la face externe de la pellicule 41, de manière analogue au mode de réalisation de la figure 2. Ainsi, le fluide sortant par la préouverture 45 pourra imprégner le tampon applicateur 46.

Sur les figures 18 à 18B, le dispositif est constitué d'une bulle creuse 200 définissant à l'intérieur une réserve 203 pour le produit à distribuer, ladite bulle 200 présentant localement une déformation 205 bombée vers l'extérieur et d'épaisseur moindre, pour constituer une zone affaiblie de préouverture par éclatement. Bien entendu, la bulle 200 pourrait être obtenue par assemblage de deux pellicules hémisphériques thermosoudées ensembles de manière définitive le long de leur bord équatorial commun.

En comprimant la bulle 200 avec les doigts D, on provoque l'éclatement de la zone affaiblie 205, pour l'éjection du produit en direction de la flèche F.

Les figures 19A à H représentent diverses formes pour la réserve et le support environnant. Sur les figures 19A à D, le support présente une forme sensiblement carrée, alors que la réserve présente respectivement une forme circulaire, carrée, triangulaire, et irrégulière en vue de dessus. Sur les figures 19E à G, le support a une forme circulaire, alors que les réserves ont respectivement une forme circulaire, carrée et triangulaire. Sur la figure 19H, le support a une forme triangulaire et la réserve a une forme circulaire. Toutefois, les formes respectives du support et de la réserve peuvent varier d'une manière quelconque, sans sortir du cadre de l'invention tel que défini dans les revendications.

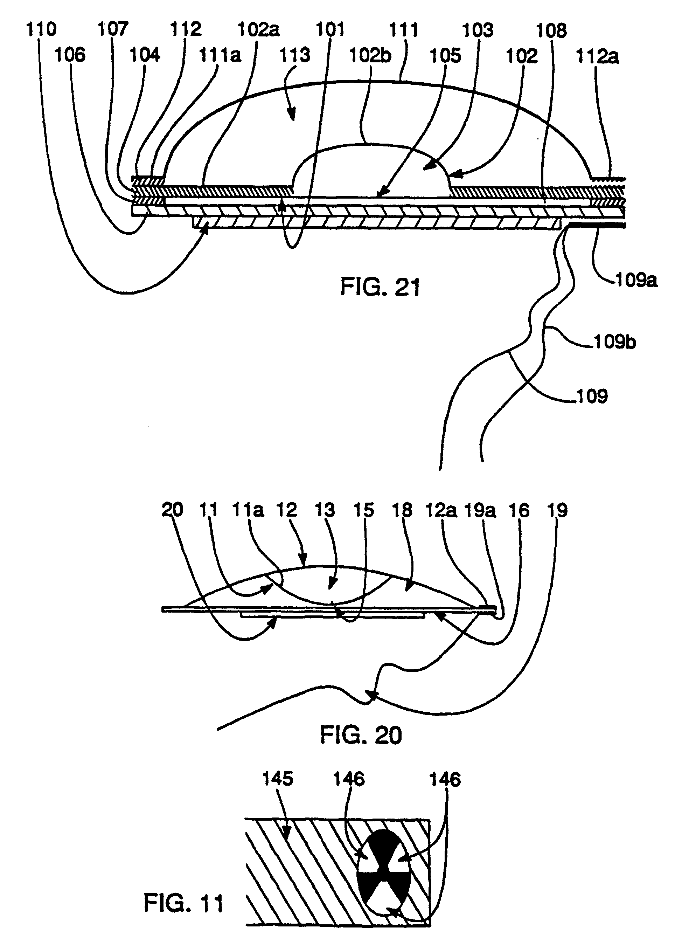

En se référant maintenant aux figures 20 et 21, on va décrire deux modes de réalisation particuliers pour un dispositif d'application de tatouage temporaire adhésif.

La figure 20 est identique à la figure 3, à l'exception d'une couche d'encre 20 pour tatouage temporaire adhésif, qui est déposée sur la surface externe du tampon applicateur 16. Lorsqu'une pression est exercée sur la pellicule 12 pour écraser la réserve 13, la préouverture 15 éclate, ce qui libère le liquide dans l'espace intermédiaire 18. Le liquide éjecté se répartit alors de manière homogène sur toute la surface interne du tampon absorbant 16, ce dernier, une fois humidifié, permettant le transfert de motifs constitués par l'encre 20 sur toute surface choisie.

Le film protecteur 19 permet d'isoler l'encre pour décalcomanie adhésive 20 de tout élément extérieur détériorant.

Dans la variante de la figure 21, les éléments identiques ou similaires à ceux illustrés sur la figure 2 portent les mêmes chiffres de référence augmentés d'une centaine. La zone hachurée 104 indique la liaison permanente entre la région périphérique 102a de la pellicule 102 et la pellicule inférieure 101.

De manière analogue à la figure 20, une couche d'encre pour décalcomanie adhésive 110 est positionnée sur la surface externe du tampon absorbant 106, et un film protecteur 109 est fixé par une patte 109a sur un bord transversal du tampon absorbant 106, pour servir de point d'articulation au film souple 109, qui peut être par exemple un film transparent. Ce film protecteur 109 est destiné à recouvrir l'encre 110, et comporte sur sa face inférieure une couche adhésive contre laquelle est plaquée une pellicule de protection pelable 109b. Une fois que le motif constitué de l'encre 110 a été transféré sur une surface donnée, on peut retirer la pellicule de protection provisoire 109b du film protecteur 109, et plaquer ce film protecteur 109 par sa face comportant la couche adhésive sur le motif imprimé, pour retirer immédiatement ce motif de la surface où il a été appliqué, et permettre son repositionnement sur une autre surface.

Le dispositif de la figure 21 comporte en outre une pellicule supplémentaire en plastique 111, de forme générale convexe tournée vers l'extérieur, dont le bord périphérique 111a est soudé de manière permanente en 112 à la face supérieure de la pellicule 102, une zone dudit bord périphérique 111a étant relié par une soudure fragilisée 112a à la pellicule 102, pour permettre l'éclatement du volume d'air 113 compris entre les pellicules 111 et 102, lorsqu'une pression est exercée sur la pellicule supplémentaire 111.

Lorsque le dispositif de la figure 21 est utilisé, on escamote d'abord le film protecteur 109 avec sa pellicule 109b, on applique la couche d'encre 110 contre la surface où elle doit être transférée, on exerce une pression sur la pellicule 111, ce qui provoque instantanément l'éclatement du volume d'air 113 par la soudure fragilisée 112a, puis l'écrasement de la réserve de liquide 103, qui éclate à son tour par la préouverture 105, ce qui provoque l'humidification du tampon applicateur 106, et libère ainsi l'encre pour décalcomanie adhésive 110 sur la surface choisie.

On notera que les pellicules utilisées dans le dispositif de l'invention ne sont pas obligatoirement en plastique, mais peuvent être en métal ou en matériaux composites.

Le support plan qui entoure la réserve, dans certains modes de réalisation, peut servir pour imprimer des informations ou des motifs. Le dispositif peut être conçu globalement transparent, opaque ou coloré.

Bien que tous les dispositifs illustrés ne comportent qu'une seule réserve, on pourrait prévoir plusieurs réserves identiques ou différentes sur un même dispositif.

La zone de préouverture sera déterminée de façon à éclater à partir d'un certain seuil de résistance, suffisamment élevé pour garantir l'intégrité de la réserve pendant son transport et son stockage, et suffisamment faible pour pouvoir être atteint par une pression manuelle.

Encore un autre avantage de l'invention est que son utilisation peut s'effectuer à l'aide d'une seule main.

Le support du dispositif peut également être percé pour pouvor être suspendu à un moyen d'accrochage, en vue de sa vente.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention tel que défini dans les revendications.

## Revendications

1. Dispositif pour diffuser une ou plusieurs doses de produit fluide, chaque dose étant contenue dans une réserve étanche (23, 33, 123, 133, 143, 153, 163, 173) qui est définie par une enveloppe au moins partiellement en matière souple déformable, de façon qu'une pression d'écrasement (P) exercée sur 1a réserve provoque l'éclatement de l'enveloppe pour libérer le produit contenu dans la réserve, l'enveloppe étant pourvue localement d'au moins une zone d'affaiblissement formant une pré-ouverture (25, 35, 125, 135, 145, 155, 165, 175), de façon à provoquer l'éclatement instantané de l'enveloppe au niveau de la pré-ouverture, le flux de produit (F) étant ainsi canalisé par la pré-ouverture dans une orientation prédéterminée, l'enveloppe comportant deux pellicules (21, 31, 121, 131, 141, 151, 161, 171 ; 22, 32, 122, 132, 142, 152, 162, 172) en matière plastique ou analogue, en métal, ou en matériaux composites, hermétiquement liées, par exemple par collage ou thermosoudage, l'une à l'autre dans une région périphérique pour définir entre elles la réserve contenant 1e produit, **caractérisé par le fait que** 1a région périphérique de liaison comporte localement au moins une zone de thermosoudure fragilisée (25, 35, 125, 135, 145, 155, 165, 175) formant la préouverture précitée, alors que le reste de la région périphérique de liaison est une thermosoudure définitive.

2. Dispositif selon la revendication 1, **caractérisé par le fait que** la zone de thermosoudure fragilisée est définie entre une portion périphérique en forme de languette (21a, 32c, 121a, 132a, 142a, 152a, 162a, 172a) de l'une des pellicules et une portion correspondante (22b, 31, 122, 131a, 141a, 151a, 161a, 171a) de l'autre pellicule, lesdites portions étant superposées et s'étendant sensiblement à distance du bord périphérique de la réserve.

3. Dispositif selon la revendication 2, **caractérisé par le fait que** la languette (21a, 121a) fait saillie à l'intérieur de la réserve (23, 123) et est thermosoudée de manière détachable, à une portion correspondante (22b, 122) de l'autre pellicule, qui présente un orifice (25a, 125a) pour diffuser le produit à l'extérieur, lorsque la languette s'est détachée sous l'effet d'une pression d'écrasement de la réserve.

4. Dispositif selon la revendication 2, **caractérisé par le fait que** la languette (32c, 132a, 142a, 152a, 162a, 172a) fait saillie à l'extérieur de la réserve (33, 133, 143, 153, 163, 173) et est thermosoudée de manière détachable à une portion correspondante (31, 131a, 141a, 151a, 161a, 171a) de l'autre pellicule, à l'exception de deux bords latéraux de la languette et, le cas échéant, de l'extrémité distale de la languette, qui sont thermosoudés de manière définitive à l'autre pellicule pour définir au moins un pré-canal d'ouverture de la réserve.

5. Dispositif selon la revendication 4, **caractérisé par le fait que** le bord d'extrémité distale (165a, 175a) de la languette est soudé de manière détachable pour définir au moins un pré-canal (165, 175) ouvrable en bout pour la sortie du produit.

6. Dispositif selon la revendication 5, **caractérisé par le fait que** la languette (162a) se prolonge au-delà de la portion correspondante (161a) de l'autre pellicule, de manière à pouvoir replier et fixer de manière détachable la portion saillante (169) de la languette à l'autre pellicule, afin d'isoler le passage de sortie du produit des éléments contaminants extérieurs, avant son utilisation.

7. Dispositif selon la revendication 4, **caractérisé par le fait que** le bord d'extrémité distale de la languette (32c, 132a, 142a, 152a) est thermosoudé de manière définitive à l'autre pellicule, pour définir au moins un pré-canal fermé (35, 135, 145, 155), et au moins un orifice (35a, 135a, 145a, 146, 155a) est ménagé à travers la languette et/ou la portion correspondante de l'autre pellicule pour la sortie du produit, lorsque la thermosoudure fragilisée se sera détachée sous l'effet de la pression exercée sur la réserve.

8. Dispositif selon la revendication 7, **caractérisé par le fait que** chaque orifice de sortie (35a, 135a, 155a) comporte un embout saillant pour définir une buse de projection du produit.

9. Dispositif selon la revendication 7, **caractérisé par le fait que** chaque orifice (145a) est recouvert d'une pellicule de protection (149) escamotable sous l'effet de la pression du produit sortant par l'orifice, afin d'isoler l'orifice des éléments contaminants extérieurs avant l'utilisation.

10. Dispositif selon l'une quelconque des revendications 7 à 9, **caractérisé par le fait que** l'orifice de sortie est constitué d'une pluralité de micro-orifices (146), disposés par exemple en étoile, pour pulvériser le produit à sa sortie.

11. Dispositif selon l'une quelconque des revendications 4 à 10, **caractérisé par le fait que** la languette (172a) comporte au moins une ligne intermédiaire de soudure définitive (175b) avec l'autre pellicule entre ses bords latéraux thermosoudés de manière définitive, ladite ligne de soudure intermédiaire étant sensiblement parallèle aux dits bords latéraux pour définir une pluralité de pré-canaux (175) de diffusion du produit.

12. Dispositif selon l'une quelconque des revendications précédentes, **caractérisé par le fait que** la réserve (143, 153, 163, 173) se présente sous la forme générale d'une bulle creuse contenant le produit à diffuser.

13. Dispositif selon l'une quelconque des revendications 1 à 11, **caractérisé par le fait que** les deux pellicules formant la réserve sont sensiblement planes et superposées, au moins l'une d'entre elles comportant une région bombée vers l'extérieur (22b, 32b) obtenue par exemple par moulage, pour définir la réserve précitée avec l'autre pellicule, la région périphérique (22a, 32a) des pellicules autour de ladite région bombée s'étendant sensiblement dans un même plan pour définir une surface périphérique de support et de préhension du dispositif.

14. Dispositif selon la revendication 13, **caractérisé par le fait que** les deux pellicules comportent une ligne de pliage commune passant au droit de la pré-ouverture de la réserve, de façon qu'en exerçant une pression sur la surface périphérique de support du dispositif, de part et d'autre de la ligne commune de pliage, la réserve soit écrasée par pincement entre les deux parties repliées du dispositif, ce qui provoque la diffusion du produit par la pré-ouverture située sur l'arête du dièdre formé par les deux parties repliées du dispositif.

15. Dispositif selon la revendication 13 ou 14, **caractérisé par le fait qu'**une couche de matière formant un tampon applicateur, est liée à ladite surface de support, de manière à imprégner le tampon applicateur avec le produit lors de l'éclatement de la réserve par la pré-ouverture, le tampon applicateur servant à appliquer le produit sur toute surface choisie.

16. Dispositif selon la revendication 15, **caractérisé par** un espace libre intercalaire entre le tampon applicateur et la pellicule en vis-à-vis pour permettre au produit libéré par la réserve de se répartir de manière homogène sur toute la surface du tampon applicateur.

17. Dispositif selon la revendication 15 ou 16, **caractérisé par le fait qu'**il comporte un film protecteur escamotable lié à l'un des bords du tampon applicateur sur sa face externe, pour le recouvrir et l'isoler de tout élément extérieur pouvant le détériorer et/ou le contaminer.

18. Dispositif selon l'une quelconque des revendications 15 à 17, **caractérisé par le fait qu'**une encre pour tatouage temporaire adhésif est positionnée sur la face externe du tampon applicateur, de sorte qu'une pression exercée par l'utilisateur sur la réserve provoque l'éclatement de l'enveloppe par la préouverture, ce qui génère l'humidification du tampon applicateur et le transfert instantané du tatouage temporaire adhésif sur la surface choisie.

19. Dispositif selon les revendications 17 et 18 prises en combinaison, **caractérisé par le fait que** le film protecteur est muni sur sa face externe d'une couche de matière adhésive recouverte d'une pellicule de protection pelable.

20. Dispositif selon la revendication 18 ou 19, **caractérisé par le fait qu'**il comporte une pellicule supplémentaire hermétiquement liée à la périphérie de la surface de support, du côté opposé à la pré-ouverture, de façon à définir un volume d'air entre ladite pellicule supplémentaire et la réserve, ladite pellicule supplémentaire comportant localement une zone périphérique de liaison affaiblie, de façon qu'une pression d'écrasement exercée sur ladite pellicule supplémentaire provoque successivement ou simultanément l'éclatement sonore du volume d'air au niveau de la zone de liaison affaiblie et l'éclatement de la réserve par la préouverture.

21. Dispositif selon l'une quelconque des revendications 15 à 20, **caractérisé par le fait que** ledit tampon applicateur est hermétiquement fixé à la périphérie de l'une des pellicules de l'enveloppe.

22. Dispositif selon l'une quelconque des revendications précédentes, **caractérisé par le fait que** la réserve peut contenir un produit liquide, crémeux, gélatineux ou gazeux.

## Claims

1. Device for distributing one or several doses of fluid product, each dose being contained in a sealed reserve (23, 33, 123, 133, 143, 153, 163, 173) which is defined by an envelope made at least partly from flexible, ductile material, so that a crushing pressure (P) exerted on the reserve causes the envelope to burst to release the product contained in the reserve, the envelope being provided locally with at least one weakening area forming a pre-opening (25, 35, 125, 135, 145, 155, 165, 175) so as to cause instant bursting of the envelope at the pre-opening, the flow of product (F) being channelled in this way by the pre-opening in a pre-determined direction, the envelope comprising two films (21, 31, 121, 131, 141, 151, 161, 171; 22, 32, 122, 132, 142, 152, 162, 172) in plastic material or similar, in metal, or in composite material, hermetically connected to each other, for example by sticking or heat sealing, in a peripheral area to define the reserve containing the product between them, **characterised by** the fact that the peripheral connecting area comprises in locally at least one weakened heat seal area (25, 35, 125, 135, 145, 155, 165, 175) forming the pre-opening indicated above, whereas the rest of the peripheral connecting area is a definite heat seal.

2. Device according to claim 1, **characterised by** the fact that the weakened heat seal area is defined between a peripheral section in the form of a tongue (21a, 32c, 121a, 132a, 142a, 152a, 162a, 172a) of one of the films and a corresponding section (22b, 31, 122, 131a, 141a, 151a, 161a, 171a) of the other film, these sections being superimposed and extending more or less away from the peripheral edge of the reserve.

3. Device according to claim 2, **characterised by** the fact that the tongue (21a, 121a) projects inside the reserve (23, 123) and is heat sealed in a detachable way to a corresponding section (22b, 122) of the other film, which has an opening (25a, 125a) to distribute the product towards the outside, when the tongue is detached under the effect of a reserve crushing pressure.

4. Device according to claim 2, **characterised by** the fact that the tongue (32c, 132a, 142a, 152a, 162a, 172a) projects outside the reserve (33, 133, 143, 153, 163, 173) and is heat sealed in a detachable way to a corresponding section (31, 131a, 141a, 151a, 161a, 171a) of the other film, with the exception of two lateral edges of the tongue and, if necessary, the terminal end of the tongue, which are heat sealed in a definite way to the other film to define at least one reserve opening pre-channel.

5. Device according to claim 4, **characterised by** the fact that the edge of the terminal end (165a, 175a) of the tongue is sealed in a detachable way to define at least one pre-channel (165, 175) which can be opened at the end for the product outlet.

6. Device according to claim 5, **characterised by** the fact that the tongue (162a) extends beyond the corresponding section (161a) of the other film, so as to be able to fold and fix the projecting section (169) of the tongue in a detachable way to the other film, in order to isolate the product outlet passage from external contaminating elements before its use.

7. Device according to claim 4, **characterised by** the fact that the edge of the terminal end of the tongue (32c, 132a, 142a, 152a) is heat sealed in a definite way to the other film to define at least one closed pre-channel (35, 135, 145, 155) and at least one opening (35a, 135a, 145a, 146, 155a) is arranged through the tongue and/or the corresponding section of the other film for the product outlet, when the weakened heat seal is detached under the effect of the pressure exerted on the reserve.

8. Device according to claim 7, **characterised by** the fact that each outlet opening (35a, 135a, 155a) comprises a projecting tip to define a nozzle for projecting the product.

9. Device according to claim 7, **characterised by** the fact that each opening (145a) is covered with a protective film (149) which can be retracted under the effect of the pressure of the product coming out of the opening, in order to isolate the opening from external contaminating elements before use.

10. Device according to any of claims 7 to 9, **characterised by** the fact that the outlet opening is made of a multiplicity of micro-openings (146) arranged in a star for example, to spray the product at the outlet.

11. Device according to any of claims 4 to 10, **characterised by** the fact that the tongue (172a) comprises at least one definitely sealed intermediate line (172b) with the other film between its lateral edges heat sealed in a definite way, this intermediate sealing line being more or less parallel to these lateral edges to define a multiplicity of pre-channels (175) for distribution of the product.

12. Device according to any of the previous claims, **characterised by** the fact that the reserve (143, 153, 163, 173) is in the general shape of a hollow bubble containing the product to be distributed.

13. Device according to any of claims 1 to 11, **characterised by** the fact that the two films forming the reserve are more or less flat and superimposed, at least one of them comprising an area bulging towards the outside (22b, 32b) obtained for example by molding, to define the reserve indicated above with the other film, the peripheral area (22a, 32a) of the films around this bulging area being more or less in the same plane to define a peripheral device gripping and support surface.

14. Device according to claim 13, **characterised by** the fact that the two films comprise a joint folding line passing at right angles to the pre-opening of the reserve, so that by exerting pressure on the peripheral support surface of the device, from one side to the other of the joint folding line, the reserve is crushed by pinching between the two folded parts of the device, which causes distribution of the product by the pre-opening situated on the ridge of the dihedral formed by the two folded parts of the device.

15. Device according to claim 13 or 14, **characterised by** the fact that a layer of material forming an applicator pad is connected to this support surface, so as to impregnate the applicator pad with the product on bursting of the reserve by the pre-opening, the applicator pad serving to apply the product over the whole of the selected surface.

16. Device according to claim 15, **characterised by** a free space between the applicator pad and the film opposite to allow the product released by the reserve to spread evenly over the whole surface of the applicator pad.

17. Device according to claim 15 or 16, **characterised by** the fact that it comprises a protective removable film connected to one of the edges of the applicator pad on its external face, to cover and isolate it from any external element which could damage and/or contaminate it.

18. Device according to any of claims 15 to 17, **characterised by** the fact that ink for temporary adhesive tattooing is positioned on the external face of the applicator pad, so that when pressure is exerted by the user on the reserve it causes bursting of the envelope by the pre-opening, which causes humidification of the applicator pad and instant transfer of the temporary adhesive tattooing on the selected surface.

19. Device according to claims 17 and 18 taken together, **characterised by** the fact that the protective film has a layer of adhesive material over its external face covered with a protective film which can be peeled.

20. Device according to claim 18 or 19, **characterised by** the fact that it comprises an additional film hermetically connected to the periphery of the support surface, on the side opposite the pre-opening, so as to define a volume of air between this additional film and the reserve, this additional film comprising in places a weakened peripheral connecting area, so that a crushing pressure exerted on this additional film subsequently or simultaneously causes the resounding break out of the volume of air in the weakened connecting area and the bursting of the reserve by the pre-opening.

21. Device according to any of claims 15 to 20, **characterised by** the fact that this applicator pad is hermetically fixed to the periphery of one of the films of the envelope.

22. Device according to any of the previous claims, **characterised by** the fact that the reserve may contain a liquid, creamy, gelatinous or gaseous product.

## Patentansprüche

1. Vorrichtung zum Verbreiten einer Dosis oder mehrerer Dosen eines fluiden Produkts, wobei jede Dosis in einem dichten Vorratsbehälter (23, 33, 123, 133, 143, 153, 163, 173) enthalten ist, der aus einer Umhüllung gebildet ist, welche wenigstens teilweise aus einem elastischen, verformbaren Material besteht, so dass ein auf den Vorratsbehälter ausgeübter Stauchdruck (P) das Aufplatzen der Umhüllung hervorruft, um das in dem Vorratsbehälter enthaltene Produkt freizusetzen, wobei die Umhüllung örtlich mit wenigstens einer Schwächungszone versehen ist, die eine. Voröffnung (25, 35, 125, 135, 145, 155, 165, 175) bildet, so dass das schlagartige Aufplatzen der Umhüllung bei der Voröffnung hervorgerufen wird, wobei infolgedessen der Fluss (F) des Produkts durch die Voröffnung in eine vorbestimmte Richtung kanalisiert wird, wobei die Umhüllung zwei Folien (21, 31, 121, 131, 141, 151, 161, 171; 22, 32, 122, 132, 142, 152, 162, 172) aus Kunststoff oder dergleichen, aus Metall oder aus einem Verbundstoff umfasst, die zum Beispiel durch Kleben oder Wärmeschweißung in einer Umfangsrichtung dicht miteinander verbunden sind, um zwischen sich den Vorratsbehälter zu bilden, der das Produkt enthält, **dadurch gekennzeichnet, dass** der Umfangsbereich der Verbindung örtlich wenigstens eine geschwächte Wärmeschweißzone (25, 35, 125, 135, 145, 155, 165, 175) aufweist, die die vorgenannte Voröffnung umfasst, während der Rest des Umfangsbereichs der Verbindung eine feste Wärmeschweißung ist.

2. Vorrichtung nach Anspruch 1, **dadurch gekennzeichnet, dass** die geschwächte Wärmeschweißzone zwischen einem laschenförmigen Umfangsabschnitt (21a, 32c, 121a, 132a, 142a, 152a, 162a, 172a) einer der Folien und einem entsprechenden Abschnitt (22b, 31, 122, 131a, 141a, 151a, 161a, 171a) der anderen Folie gebildet ist, wobei die Abschnitte übereinanderliegen und sich im wesentlichen in einem Abstand des Umfangsrandes des Vorratsbehälters erstrecken.

3. Vorrichtung nach Anspruch 2, **dadurch gekennzeichnet, dass** die Lasche (21a, 121a) ins Innere des Vorratsbehälters (23, 123) ragt und lösbar mit einem entsprechenden Abschnitt (22b, 122) der anderen Folie verbunden ist, die eine Öffnung (25a, 125a) aufweist, um das Produkt nach außen zu verbreiten, wenn sich die Lasche durch einen Stauchdruck des Vorratsbehälters abgelöst hat.

4. Vorrichtung nach Anspruch 2, **dadurch gekennzeichnet, dass** die Lasche (32c, 132a, 142a, 152a, 162a, 172a) von dem Vorratsbehälter (33, 133, 143, 153, 163, 173) nach außen hervorragt und mit Außnahme von zwei seitlichen Rändern der Lasche, und gegebenenfalls dem äußeren Ende der Lasche, die fest mit der anderen Folie wärmeverschweißt sind, an einem entsprechenden Abschnitt (31, 131a, 141a, 151a, 161a, 171a) der anderen Folie lösbar wärmeverschweißt ist, um wenigstens einen Öffnungs-Vorkanal des Vorratsbehälters zu bilden.

5. Vorrichtung nach Anspruch 4, **dadurch gekennzeichnet, dass** der Rand des distalen Endes (165a, 175a) der Lasche lösbar verschweißt ist, um wenigstens einen vom Ende öffnenbaren Vorkanal (165, 175) für den Austritt des Produkts zu bilden.

6. Vorrichtung nach Anspruch 5, **dadurch gekennzeichnet, dass** sich die Lasche (162a) über den entsprechenden Abschnitt (161a) der anderen Folie hinaus so fortsetzt, dass der überragende Abschnitt (169) der Lasche um die andere Folie umgeklappt und mit ihr lösbar befestigt werden kann, um die Austrittsdurchführung des Produkts von seiner Verwendung von äußeren Verunreinigungselementen zu isolieren.

7. Vorrichtung nach Anspruch 4, **dadurch gekennzeichnet, dass** der Rand des äußeren Endes der Lasche (32c, 132a, 142a, 152a) fest an die andere Folie wärmegeschweißt ist, um wenigstens einen geschlossenen Vorkanal (35, 135, 145, 155) zu bilden, und wenigstens eine Öffnung (35a, 135a, 145a, 146, 155a) ist quer durch die Lasche und/oder den entsprechenden Abschnitt der anderen Folie zum Austritt des Produkts ausgespart, da sich die abgeschwächte Wärmeschweißung unter der Wirkung des auf den Vorratsbehälter ausgübten Drucks abgelöst haben wird.

8. Vorrichtung nach Anspruch 7, **dadurch gekennzeichnet, dass** jede Austrittsöffnung (35a, 135a, 155a) ein hervorragendes Ansatzstück aufweist, um eine Produkt Strahldüse zu bilden.

9. Vorrichtung nach Anspruch 7, **dadurch gekennzeichnet, dass** jede Öffnung (145a) mit einer Schutzfolie (149) abgedeckt ist, die durch den Druck des durch die Öffnung austretenden Produkts entfernt werden kann, um die Öffnung vor der Verwendung von äußeren Verunreinigungselementen zu isolieren.

10. Vorrichtung nach einem der Ansprüche 7 bis 9, **dadurch gekennzeichnet, dass** die Austrittsöffnung aus einer Mehrzahl von Mikro-Öffnungen (146) gebildet ist, die zum Beipiel sternförmig angeordnet sind, um das Produkt bei seinem Austritt zu zerstäuben.

11. Vorrichtung nach einem der Ansprüche 4 bis 10, **dadurch gekennzeichnet, dass** die Lasche (172a) zwischen ihren fest wärmeverschweißten seitlichen Rändern wenigstens eine feste mittlere Verschweißungslinie (175b) mit der anderen Folie aufweist, um eine Mehrzahl von Vorkanälen (175) zur Verbreitung des Produkts zu bilden, wobei die mittlere Verschweißungslinie im wesentlichen parallel zu den seitlichen Rändern verläuft.

12. Vorrichtung nach einem der vorgenannten Ansprüche, **dadurch gekennzeichnet, dass** der Vorratsbehälter (143, 153, 163, 173) die allgemeine Form einer Hohlkugel aufweist, die das zu verbreitende Produkt enthält.

13. Vorrichtung nach einem der Ansprüche 1 bis 11, **dadurch gekennzeichnet, dass** die zwei Folien, die den Vorratsbehälter bilden, im wesentlichen flach sind und aufeinander liegen, wobei wenigstens eine von ihnen einen Bereich (22b, 32b) aufweist, der nach außen gewölbt und zum Beispiel durch Gießen gebildet ist, um mit der anderen Folie den genannten Vorratsbehälter zu bilden, wobei sich der Umfangsbereich (22a, 32a) der Folien um den gewölbten Bereich im wesentlichen in derselben Ebene erstreckt, um eine Umfangshalte- und Greiffläche der Vorrichtung zu bilden.

14. Vorrichtung nach Anspruch 13, **dadurch gekennzeichnet, dass** die zwei Folien eine gemeinsame Faltlinie aufweisen, die gerade durch die Voröffnung des Vorratsbehälters führt, so dass der Vorratsbehälter durch Zusammenklemmen zwischen den beiden umgebogenen Bereichen der Vorrichtung zusammengedrückt wird, wenn beidseits der gemeinsamen Faltlinie ein Druck auf die Umfangshaltefläche der Vorrichtung ausgeübt wird, wodurch die Verbreitung des Produkts durch die Voröffnung bewirkt wird, die auf der Kante des Dieders angeordnet ist, der durch die beiden umgebogenen Bereiche der Vorrichtung gebildet ist.

15. Vorrichtung nach einem der Ansprüche 13 oder 14, **dadurch gekennzeichnet, dass** eine Materialschicht, die ein Auftragungskissen bildet, derart mit der Haltefläche verbunden ist, dass das Auftragungskissen beim Aufplatzen des Vorratsbehälters durch die Voröffnung mit dem Produkt getränkt wird, wobei das Auftragungskissen dazu dient, das Produkt auf die gesamte ausgewählte Oberfläche zu applizieren.

16. Vorrichtung nach Anspruch 15, **gekennzeichnet durch** einen zwischen dem Auftragungskissen und der gegenüberliegenden Folie angeordneten freien Raum, um zu ermöglichen, dass sich das von dem Vorratsbehälter freigesetzte Produkt gleichmäßig auf der gesamten Oberfläche des Auftragungskissens verteilt.

17. Vorrichtung nach Anspruch 15 oder 16, **dadurch gekennzeichnet, dass** sie eine entfernbare Schutzschicht aufweist, die mit ihrer äußeren Seite mit einem der Ränder des Auftragungskissens verbunden ist, um es abzudecken und es von jedem äußeren Element zu isolieren, das es beschädigen und/oder verunreinigen könnte.

18. Vorrichtung nach einem der Ansprüche 15 bis 17, **dadurch gekennzeichnet, dass** eine Tinte für eine vorübergehende Klebetätowierung auf der äußeren Oberfläche des Auftragungskissens so angeordnet ist, dass ein von dem Anwender auf den Vorratsbehälter ausgeübter Druck durch die Voröffnung das Aufplatzen der Umhüllung verursacht, wodurch die Befeuchtung des Auftragungskissens und die sofortige Übertragung der vorübergehenden Klebetätowierung auf die ausgewählte Oberfläche erzeugt wird.

19. Vorrichtung nach den Ansprüchen 17 und 18, **dadurch gekennzeichnet, dass** die Schutzschicht auf ihrer äußeren Seite mit einer Schicht aus klebendem Material versehen ist, die mit einer abziehbaren Schutzfolie abgedeckt ist.

20. Vorrichtung nach Anspruch 18 oder 19, **dadurch gekennzeichnet, dass** sie eine zusätzliche Folie umfasst, die von der gegenüberliegenden Seite bis zu der Voröffnung dicht mit dem Umfang der Haltefläche so verbunden ist, dass zwischen der zusätzlichen Folie und dem Vorratsbehälter ein Luftvolumen gebildet ist, wobei die zusätzliche Folie örtlich eine abgeschwächte Umfangsverbindungszone aufweist, so dass ein auf die zusätzliche Folie ausgeübter Stauchdruck nacheinander oder gleichzeitig das laute Aufplatzen des Luftvolumens bei der geschwächten Verbindungszone und das Aufplatzen des Vorratsbehälters durch die Voröffnung hervorruft.

21. Vorrichtung nach einem der Ansprüche 15 bis 20, **dadurch gekennzeichnet, dass** das Auftragungskissen dicht an dem Umfang von einer der Folien der Umhüllung befestigt ist.

22. Vorrichtung nach einem der vorgenannten Ansprüche, **dadurch gekennzeichnet, dass** der Vorratsbehälter ein flüssiges, cremiges, Gelatine-artiges oder gasförmiges Produkt enthält.
